# FASCICULE DE BREVET EUROPEEN

(11) **EP 1 377 316 B1**
(45) Date de publication et mention de la délivrance du brevet: **04.06.2008**
(21) Numéro de dépôt: 02732804.6
(22) Date de dépôt: 11.04.2002
(51) Int. Cl.: A61K 47/44, A61K 9/08, A61K 31/704, A61P 27/02

(54) **COMPOSITION PHARMACEUTIQUE A BASE DE AZALIDES POUR APPLICATION LOCALE EN OPHTALMOLOGIE**
PHARMAZEUTISCHE ZUSAMMENSETZUNG AUF DER BASIS VON AZALIDEN FÜR LOKALE ANWENDUNG IN DER OPHTALMOLOGIE
PHARMACEUTICAL COMPOSITION BASED ON AZALIDES FOR TOPICAL APPLICATION IN OPHTHALMOLOGY

(30) Priorité: 12.04.2001 FR 0105114
(43) Date de publication de la demande: 07.01.2004
(73) Titulaire: Laboratoires THEA, 63100 Clermont-Ferrand (FR)
(72) Inventeur: LUYCKX, Jacques, F-63112 Ceyrat (FR); PILOTAZ, Frédéric, F-63400 Chamalieres (FR)
(74) Mandataire: Thibon-Littaye, Annick
(86) Numéro de dépôt international: PCT/FR2002/001263
(87) Numéro de publication internationale: WO 2002/083178

(56) Documents cités:
- EP-A- 0 925 789
- WO-A-00/57866
- GB-A- 1 152 644
- ARTHUR H.KIBBE: "Handbook of pharmaceutical excipients, 3rd edition" , ALPHA , WASHINGTON XP002187260 page 329 -page 331
- ARTHUR H.KIBBE: "Handbook of pharmaceutical excipients, 3rd edition" , ALPHA , WASHINGTON XP002187261 page 362 -page 364

## Description

La présente invention concerne une nouvelle composition pharmaceutique pour la prévention et le traitement des infections oculaires qui convient pour être utilisée en application locale dans l'oeil. En tant que principe actif essentiel, elle comporte un agent antibiotique de la famille des macrolides choisi parmi les azalides, qu'elle propose de présenter en forme pharmaceutique de collyre, distribuable goutte à goutte.

Les macrolides sont connus depuis déjà longtemps pour être des antibiotiques particulièrement efficaces, y compris contre les infections oculaires. Il s'agit de composés d'origine naturelle ou plus ou moins synthétique dont la formule comporte un hétérocycle intégrant une fonction lactone, généralement associé à des groupes saccharidiques.

L'exemple de base des macrolides est l'érythromycine, où l'hétérocycle à fonction lactone de la molécule est déjà à 14 chaînons. L'efficacité antibiotique a été vérifiée non seulement pour l'érythromycine proprement dite, mais aussi pour de nombreux composés qui dérivent de sa structure par des substituants divers sur le cycle principal. On peut citer à ce titre la clarithromycine, la roxithromycine, la dirithromycine, pour les cycles à 14 chaînons, mais aussi les composés tels que la spiramycine ou la josamycine où la cyclisation couplant deux substituants conduit à un cycle lactone à 16 chaînons.

Les azalides représentent un groupe plus récent dans la classe des macrolides. Ils dérivent de la structure de base de l'érythromycine par insertion d'un atome d'azote intracyclique dans le cycle à fonction lactone. Le chef de file de cette sous-classe de macrolides antibiotiques est l'azithromycine, correspondant à la *N-*méthyl-11-aza-10-désoxo-10-dihydro-érythromycine. Dans ce composé, les groupes hydroxyle des positions 11 et 12 de l'érythromycine n'étant pas substitués, le cycle à fonction lactone devient à 15 chaînons. Il est particulièrement courant sous la forme du dihydrate, ce qui toutefois n'a rien de limitatif, le composé pouvant aussi être, de manière classique, sous la forme de tout sel biologiquement compatible, comme dans le cas d'ailleurs des autres composés de la classe.

Le document GB-A-1 152 644 décrit des compositions d'érythromycine dans un véhicule huileux. Un des véhicules cités est notamment constitué de triglycérides d'acides gras. Ces compositions sont destinées au traitement des inflammations de la glande mammaire chez les bovins, par injection par voie parentérale.

Comme pour tous composés utiles en ophtalmologie, l'industrie pharmaceutique a travaillé à permettre d'utiliser les macrolides en application locale plutôt que par administration par voie générale, et s'agissant d'une application dans l'oeil, la forme collyre vient immédiatement à l'esprit. Mais les essais en ce sens se sont heurtés à des difficultés que la présente invention vise à résoudre. On pourra en particulier se référer au document d'art antérieur constitué par la demande de brevet internationale WO 00/57866 (Insite Vision). Ce document rappelle des connaissances générales sur les propriétés des muqueuses, les maladies et agents pathogènes du ressort d'un traitement ophtalmique et les conditions d'administration des antibiotiques, et il détaille différentes formules de macrolides en s'intéressant principalement à l'azithromycine et à ses conditions d'emploi. En se montrant soucieux de traiter les maladies oculaires par application locale d'un azalide, ses inventeurs n'ont su faire autrement pour cela que de proposer de présenter l'azalide en question sous forme d'une suspension dans de l'eau additionnée d'un polymère de mise en suspension. La même critique est à porter à l'encontre des compositions sous forme de suspensions d'azithromycine qui sont décrites dans la demande de brevet européen EP 0925789 (Pfizer).

La présente invention permet d'éviter les inconvénients qui en résultent, notamment du point de vue des risques d'irritation des muqueuses de l'oeil et de la cornée. Elle vise aussi, d'une manière plus générale, à améliorer les conditions de mise en oeuvre de l'activité des composés antibiotiques de la famille des macrolides dans leurs applications en ophtalmologie. Il figure ainsi dans son propos de faciliter la pénétration de l'agent actif dans la cornée et les tissus conjonctifs de l'oeil, d'améliorer sa rétention et le temps de rémanence, de s'assurer qu'à la concentration nécessaire en fonction de la dose active à administrer à chaque instillation le produit n'entraîne pas d'irritation oculaire, de réduire le nombre d'instillations journalières et la durée du traitement, et d'une manière générale de mieux satisfaire aux différentes exigences de la pratique médicale, y compris en préservant les avantages de l'administration locale. De ce point de vue, l'invention a également en particulier pour objectif d'assurer un bon confort de traitement, de faciliter l'administration de la composition et d'éviter tout trouble de la vision qui pourrait apparaître consécutivement à une application par voie topique. L'invention vise aussi à améliorer les conditions de fabrication industrielle des médicaments, en particulier en ce qui concerne les coûts, ainsi que la durée de conservation du produit jusqu'à son utilisation.

Compte tenu de ces objectifs et d'autres qui pourront mieux se comprendre de la suite de la présente description, l'invention propose une composition pharmaceutique convenant au traitement d'infections oculaires par application locale, conforme à la revendication 1.

Elle s'applique d'une façon spécialement avantageuse aux azalides, et plus particulièrement à l'azithromycine. Ce dernier composé semble actuellement le meilleur dans l'optique de l'invention. En conséquence, toutes les particularités de l'invention qui seront précisées ci-après le seront, sauf indication contraire, en admettant que le principe actif auxquelles elles conviennent le mieux du point de vue industriel est l'azithromycine, le cas échéant en tant que composé type de la classe des azalides.

Le recours à un véhicule huileux plutôt qu'à un milieu aqueux comme il a été proposé à ce jour permet d'assurer une dissolution complète du principe actif aux concentrations utiles. On obtient un liquide qui est une vraie solution, considérée dans ce qui la distingue d'une suspension, aussi fines que puissent être les particules en suspension, alors même que la solution peut présenter des viscosités très variables. En outre, quelle que soit la technique parmi celles qui ont pu être envisagées pour mettre en solution des macrolides dans des milieux divers, on se heurte, notamment dans le cas des azalides, à des problèmes d'instabilité de la solution dont on conçoit aisément qu'elles sont particulièrement cruciales pour des compositions destinées à être utilisées en application locale dans l'oeil et qu'elles représentent aussi un frein à une industrialisation dans de bonnes conditions.

Un des principaux avantages à disposer d'une vraie solution réside dans le fait que le principe actif s'applique alors de façon homogène sur toute la surface de l'oeil, alors que dans le cas d'une suspension, le contact des particules de composé actif sur l'oeil est irrégulier. En outre, les azalides sont quasiment insolubles dans l'eau, ainsi que dans les larmes, et leurs particules solides sont donc plus facilement éliminées de l'oeil par les larmes, d'où un temps de rémanence réduit pour les compositions sous forme de suspensions aqueuses.

En association avec la solubilisation complète du macrolide dans le véhicule suivant l'invention, les propriétés inhérentes au caractère huileux du véhicule participent également à augmenter le temps de rémanence de la composition dans l'oeil. En effet, il se forme un film huileux sur la surface de l'oeil qui est moins facilement éliminé par les larmes que dans le cas d'une composition aqueuse.

La formulation de la composition suivant l'invention sous forme d'un collyre huileux présente également d'autres avantages. En particulier, elle n'engendre quasiment aucun trouble de la vision, contrairement à ce que l'on devrait attendre d'une composition qui a par ailleurs l'avantage d'un temps de rémanence amélioré du fait du véhicule huileux. Or on sait, par exemple, que d'autres formes galéniques couramment utilisées pour une application par voie topique en ophtalmologie, telles que la pommade et la crème, entraînent un trouble de la vision durant un temps relativement long après l'administration.

L'invention s'exprime également en un procédé de préparation d'une composition pharmaceutique convenant pour application locale en ophtalmologie selon la revendication 8. Ce procédé est caractérisé en ce qu'il consiste à mettre le composé antibiotique en solution dans un véhicule huileux, avantageusement en l'absence d'autres constituants, tels que ceux que l'on sait utiliser dans d'autres contextes pour favoriser la mise en solution d'un produit, avantageusement aussi sans ajouter d'agents secondaires tels que les émulsifiants, épaississants, ou conservateurs divers.

Suivant des caractéristiques secondaires de l'invention, la dissolution du composé actif dans le véhicule huileux s'effectue soit à la température ordinaire, ce qui est en général préférable, ne serait-ce que pour simplifier les conditions de mise en oeuvre du procédé et limiter son coût, soit sous léger chauffage, notamment à une température comprise entre 30 °C et 90 °C, et de préférence de l'ordre de 50 à 80 °C, ou plus particulièrement proche de 70 °C, notamment dans le cas où le composé actif est l'azithromycine. En effet, les composés actifs préconisés suivant la présente invention se révèlent, non seulement quasiment insolubles dans l'eau, mais aussi sensibles à la chaleur, et il importe d'opérer à une température à laquelle le principe actif reste stable.

Le plus souvent, on choisira un véhicule de viscosité relativement faible, afin de pouvoir également réaliser la stérilisation de la solution, dans les mêmes conditions de température, au cours d'une seconde étape du procédé consistant à opérer pour cela par filtration, et de préférence à froid comme pour la mise en solution. On observe là l'un des intérêts majeurs de l'invention, car par exemple, la solution peut être stérilisée d'une manière simple et efficace, au travers d'une membrane filtrante offrant des dimensions de pores au plus égales à 0,2 µm, et préférentiellement comprises entre 0,1 et 0,2 µm, ce qui ne pourrait être envisagé dans le cas de suspensions.

Ceci est particulièrement avantageux dans le cas présent des molécules thermosensibles que sont les macrolides, ou plus spécialement les azalides, puisque les compositions dans lesquelles elles sont contenues ne peuvent être stérilisées par les moyens classiques faisant appel à un chauffage à des températures élevées, comme par autoclave. De même, l'exposition à des radiations gamma, une autre méthode couramment utilisée pour la stérilisation de compositions comprenant certains antibiotiques, cause la dégradation des macrolides, et il est donc préférable de l'éviter.

D'une manière générale, la concentration de l'agent actif dans le véhicule huileux peut être très variable suivant le composé actif particulier, dans une gamme allant de 0,1 à 2 % en poids du poids total. Cependant, comme on a pu le vérifier tout particulièrement dans le cas de l'azithromycine, l'invention a l'avantage de conduire à des solutions stables à des concentrations de principe actif relativement élevées, ce qui conduit à préférer, pour des questions d'efficacité du traitement, des concentrations comprises entre 0,7 et 2 %, en particulier de l'ordre de 1 à 1,5 % en poids du poids total.

Dans le cadre des modes de mise en oeuvre préférés de l'invention le véhicule huileux utilisé comme excipient solvant du composé actif antibiotique répond en outre à une ou plusieurs des caractéristiques secondaires évoquées ci-après, lesquelles peuvent avantageusement être appliquées simultanément ou en toute combinaison techniquement opérante.

C'est ainsi que le véhicule huileux est choisi de préférence pour être une huile grasse d'origine végétale, mais raffinée et/ou hydrogénée et présentant une viscosité à la température ordinaire inférieure à 100 mPa.s (soit 100 cPo). On satisfait par là non seulement à des conditions appropriées pour des collyres, en ce qui concerne la commodité de distribution goutte à goutte, mais aussi à des conditions de fabrication industrielle particulièrement avantageuses.

Il est en outre souhaitable que la viscosité de la composition finale soit d'au moins 10 mPa.s à température ordinaire, notamment pour des questions de rémanence sur la cornée de l'oeil. En pratique, les viscosités préférées sont comprises entre 10 et 50 mPa.s, particulièrement de l'ordre de 20 à 40 mPa.s, du moins quand on vise à simplifier au mieux le procédé de fabrication du médicament. En effet, on favorise alors une fabrication industrielle passant par une stérilisation effectuée à froid sur la solution du macrolide dans l'huile, sans demander le moindre chauffage.

Il est évident que ceci n'empêche pas qu'il puisse être utile, pour des cas d'application particuliers, de compléter la composition pharmaceutique obtenue en y ajoutant un agent épaississant compatible avec les conditions d'administration du produit final. Le technicien en la matière saura pour cela trouver dans l'industrie des épaississants qu'il utilisera dans des conditions stériles afin d'augmenter la viscosité sans détruire la solution (citons par exemple l'acide cétylique, l'acide stéarique, ou leurs dérivés). Un effet "retard" de libération progressive et prolongée du principe actif est obtenu en modulant ainsi la viscosité de la solution, sans toutefois perdre de vue le fait que conformément à l'invention, elle doit pouvoir être facilement instillée sous forme de gouttes.

D'autres particularités avantageuses du véhicule huileux convenant à la mise en oeuvre de l'invention sont plutôt d'ordre chimique. Les exigences correspondantes sont particulièrement bien satisfaites par les huiles grasses qu'il est courant d'appeler des triglycérides à chaîne moyenne, ou "medium chain triglycerides" en anglais. Suivant la norme instaurée par la pharmacopée européenne, ces huiles sont extraites de la noix de coco et essentiellement constituées, pour au moins 95 %, par des triglycérides des acides gras saturés que sont l'acide caprylique et l'acide caprique. Alternativement elles sont donc dites constituées de triglycérides caprylique/caprique, ou de caprytate/caprate de triglycéryle.

La composition ainsi obtenue par dissolution du macrolide dans un véhicule constitué de triglycérides à chaîne moyenne est particulièrement appropriée au traitement ophtalmique par application locale, puisqu'elle permet entre autres de répondre à un des objectifs de l'invention, à savoir d'assurer que la composition présente un risque d'irritation des tissus oculaires réduit au minimum. Par irritation, on inclut tous les effets de brûlure temporaire, de picotement ou de pleurs, qui pourraient être causés par le véhicule utilisé dans la formulation ou par des composés qui lui sont associés. Ceci revêt d'autant plus d'importance que la présence de larmes dans les yeux, en conséquence d'une irritation, accroît la vitesse d'élimination de la composition antibiotique.

Il apparaît donc tout à fait avantageux d'utiliser dans le cadre de l'invention les triglycérides à chaîne moyenne, qui ont pour particularité d'être très bien définis chimiquement, et de ne pas être associés à des produits secondaires qui pourraient causer une irritation par contact avec l'oeil. De plus, compte tenu de la solubilité des macrolides antibiotiques utilisés dans ce véhicule huileux, la composition suivant l'invention présente l'avantage de ne pas nécessiter l'ajout d'additifs, tels que des polymères de mise en suspension, ce qui réduit considérablement le risque d'irritation des tissus oculaires. Pour la même raison, le solvant huileux constitué par des triglycérides à chaîne moyenne est, suivant l'invention, largement préférable à la lanoline, que l'on peut également citer pour dissoudre les macrolides, mais qui a l'inconvénient d'être irritante pour l'oeil.

Dans le cadre de la mise en oeuvre de l'invention, il résulte avantageusement de ces caractéristiques des triglycérides à chaîne moyenne une meilleure maîtrise de la composition de la formulation, une réduction des effets secondaires qui pourraient être engendrés par des additifs irritants, ainsi qu'une meilleure maîtrise de la fabrication industrielle du collyre.

Plus généralement dans le cadre de la mise en oeuvre de l'invention, les véhicules huileux sont choisis parmi les huiles grasses d'origine végétale dont on sait disposer industriellement et qui sont essentiellement constitués, notamment pour au moins 80 % (et de préférence au moins 95 %), de triglycérides d'acides gras. Il s'agit de préférence de composés dans lesquels les trois fonctions alcool du glycérol sont estérifiées par les fonctions acide d'acides gras, ces derniers étant de préférence des acides carboxyliques d'hydrocarbures saturés. En outre, le véhicule huileux utilisé comme solvant du composé actif conformément à l'invention est avantageusement sélectionné pour que de tels triglycérides le constituant essentiellement dérivent d'un ou plusieurs acides d'hydrocarbures de poids moléculaire moyen présentant une fonction acide carboxylique en bout d'une chaîne linéaire. La molécule d'acide est donc préférentiellement de longueur moyenne. De ce point de vue, elle comporte notamment de 5 à 12 atomes de carbone, et de préférence de 8 à 10 atomes de carbone, ce qui est en particulier le cas de l'acide caprylique et de l'acide caprique.

Le véhicule huileux préférentiellement utilisé pour la formulation de l'invention présente un très faible taux d'insaturation. De façon classique, le taux d'insaturation d'une huile peut être déterminé par le taux d'halogène que peuvent lier les liaisons multiples. Le taux d'halogène lié, exprimé en terme de molécules d'iode par 100 g d'huile, est connu comme le nombre d'iode. Ainsi, le véhicule huileux suivant l'invention présente de préférence un nombre d'iode inférieur ou égal à 1. Le véhicule huileux utilisé préférentiellement pour la formulation de l'invention présente par ailleurs une densité avantageusement comprise entre 0,9 et 1, et préférentiellement proche de 0,95. Suivant d'autres particularités de l'invention, son indice de réfraction est de préférence compris entre 1 et 2, préférentiellement compris entre 1,3 et 1,5, notamment de l'ordre de 1,45 et sa densité est avantageusement proche de 1, notamment comprise entre 0,9 et 1.

Selon un mode de réalisation préféré de l'invention, l'agent thérapeutique actif est l'azithromycine à une concentration préférentiellement comprise entre 0,7 et 2% en poids, et préférentiellement entre 1 et 1,5% en poids. Le véhicule huileux suivant l'invention permet de solubiliser parfaitement l'azithromycine à ces concentrations, ce qui est particulièrement avantageux par rapport aux véhicules aqueux. En effet, si on connaît de façon classique des moyens de solubiliser l'azithromycine dans des compositions aqueuses, par exemple dans un mélange acide citrique/composé salin (comme envisagé dans la demande de brevet européen EP 1 075 837 au nom de SIFI), il a été constaté par les présents inventeurs que l'azithromycine est relativement peu stable dans ce type de solutions. De façon tout à fait avantageuse, elle est au contraire très stable dans les solutions huileuses d'origine végétale selon l'invention.

Selon une formulation particulière de l'invention, la composition pharmaceutique est préférentiellement destinée à être présentée en flaconnages unidoses. On entend par là un conditionnement jetable au premier usage, contenant la quantité de produit utile pour deux instillations, une dans chaque oeil. Du fait de la stabilité des compositions de l'invention, il est alors avantageux de n'ajouter aucun additif à la solution, et notamment aucun conservateur.

Cependant, selon un autre mode de mise en oeuvre de l'invention, la composition pharmaceutique comporte un agent de conservation, comme il a été indiqué précédemment. Cette solution est en général préférée quand le produit est conditionné en flaconnages multidoses. L'agent de conservation est préférentiellement du phénoxyéthanol, de l'alcool phényléthylique, du chlorobutanol, un sel d'ammonium quaternaire, ou tout autre conservateur adapté pour un collyre. Dans ce dernier cas, lors de la préparation de la formulation, les composés d'ammonium quaternaire, tels que le chlorure de benzalkonium, sont préférentiellement incorporés à chaud et leur concentration ne dépasse préférentiellement pas 0,01% en poids. Les conservateurs du type des parabènes s'incorporent de la même manière. Il s'agit notamment de parahydroxybenzoates de méthyle et/ou de propyle et/ou de butyle.

Selon un procédé de préparation préféré de l'invention, la composition pharmaceutique est stérilisée par filtration, avantageusement sur une membrane de porosité comprise entre 0,1 et 0,2 µm. Cette opération peut être réalisée à froid, plus exactement à la température ordinaire, ou sous léger chauffage à une température de l'ordre de 30 à 40 °C, ou encore à une température plus élevée, mais restant inférieure à 80 °C, par exemple à une température de l'ordre de 70 °C. Cette dernière solution pourra être préférée notamment quand la dissolution du composé actif dans le véhicule huileux est également opérée à chaud, auquel cas on pourra se placer dans les mêmes conditions de température pour la stérilisation. Mais quand la viscosité du véhicule huileux choisi le permet, il paraît en général préférable d'effectuer la stérilisation par filtration à la température ordinaire, pour des raisons de commodité et de coût. Différents types de membranes peuvent être utilisés pour cette opération de filtration, telles que celles à base d'acétate de cellulose, de fluorure de polyvinylidène (PVDF), de polyéthersulfone (PES), de polytétrafluoréthylène (PTFE).

Pour compléter la description des caractéristiques de l'invention ainsi que celle de ses avantages et résultats, on considérera maintenant quelques exemples de préparation du médicament selon l'invention, qui bien entendu ne sont pas limitatifs. Le principe actif est présent sous forme base, sans salification des fonctions amine. Des exemples comparatifs de l'efficacité du médicament selon l'invention par rapport à deux autres médicaments à base d'azithromycine sont également fournis, ainsi qu'un exemple de pharmacocinétique oculaire pour des compositions à base d'azithromycine suivant l'invention à différentes concentrations en principe actif.

### Exemple 1

On réalise un collyre à base d'azithromycine à 1,5 % en poids, sous la formulation centésimale :

| | | |
|---|---|---|
| Azithromycine | 1,5 g | |
| Triglycérides à chaîne moyenne | Q.S.P. | 100 g |

Le véhicule huileux utilisé correspond à la définition de la pharmacopée européenne pour une huile grasse raffinée à 95% de triglycérides à chaînes moyennes dans lesquels les fonctions alcool du glycérol sont entièrement estérifiées par des acides carboxyliques d'hydrocarbures saturés dont la chaîne est linéaire et de longueur moyenne, à savoir essentiellement l'acide caprique et/ou l'acide caprylique.

Une masse de triglycérides à chaîne moyenne (TCM) correspondant à 99 % de sa masse finale normale est chauffée à 70 °C au bain-marie. La poudre d'azithromycine est dissoute dans les triglycérides sous agitation. L'ensemble est maintenu à 70 °C pendant quelques minutes, puis on laisse refroidir la solution obtenue jusqu'à la température ambiante. On ajuste alors au poids avec le TCM, et on parfait le mélange quelques minutes sous agitation.

L'azithromycine reste en solution après refroidissement, et la solution obtenue est claire et limpide. Elle est soumise à un traitement de stérilisation qui s'effectue par filtration. On procède à la température ordinaire. La filtration est réalisée sous ambiance stérile, sur un filtre à mailles de 0,2 µm fait d'une membrane de polyéthersulfone. On obtient une vraie solution, qui ne nécessite pas, comme dans le cas des formulations de macrolides en véhicule aqueux, l'addition de polymères de mise en suspension.

Des études de stabilité, conduites à 25 °C et 60 % d'humidité et à 40 °C et 75 % d'humidité, montrent que la solution obtenue reste stable dans le temps, durant une période supérieure à six mois.

### Exemple 2

On réalise un collyre à base d'azithromycine à 1 % en poids, destiné à être conditionné en flaconnages unidoses.

Sa formule centésimale est la suivante :

| | | |
|---|---|---|
| Azithromycine | 1 g | |
| Triglycérides à chaîne moyenne | Q.S.P. | 100 g |

On procède comme dans l'exemple 1 pour préparer le collyre d'azithromycine dans un véhicule de triglycérides à chaîne moyenne sauf que la concentration est portée à 1 % en poids du poids total de la composition. La solution obtenue est stérilisée par filtration comme dans l'exemple 1. Pour la fraction de triglycérides à chaîne moyenne retenue dans le présent exemple, le produit présente une viscosité de 30 mPa.s (30 cPo à 20 °C). Sa densité est de 0,95. Son indice de réfraction est égal à 1,45. Cette valeur est très proche de celle des larmes, laquelle est égale à 1,33 chez un sujet sain. Il en résulte que le risque de troubler la vue lors de l'instillation de collyre dans l'oeil est minimisé.

### Exemple 3

On réalise un collyre à base d'azithromycine à 1 % en poids, comportant un agent de conservation. Ce collyre, destiné à être conditionné en flaconnages multidoses, présente la composition suivante, exprimée en poids pour 100 g :

| | |
|---|---|
| Azithromycine | 1 g |
| Chlorure de benzalkonium | 0,01 g |
| Triglycérides à chaîne moyenne | Q.S.P. 100 g |

Une masse de triglycérides TCM correspondant à 99% de sa masse finale normale est chauffée à 70 °C au bain-marie. Les poudres d'azithromycine ainsi que de chlorure de benzalkonium sont dissoutes dans le TCM sous agitation. L'ensemble est maintenu à 70 °C pendant quelques minutes, puis on le laisse refroidir jusqu'à température ambiante. On ajuste alors au poids avec le TCM, et on parfait le mélange quelques minutes sous agitation. La solution est ensuite filtrée sous ambiance stérile, sur un filtre de 0,2 µm de type à haut débit en membrane de polyéthersulfone.

### Exemple 4

On réalise un collyre à base d'azithromycine à 0,5 % en poids, destiné à être conditionné en flaconnages unidoses, en procédant comme décrit dans l'exemple 1.

Sa formule centésimale est la suivante :

| | |
|---|---|
| Azithromycine | 0,5 g |
| Triglycérides à chaîne moyenne | Q.S.P. 100 g |

A titre comparatif, on réalise une composition aqueuse d'azithromycine à 1 % en poids, par dissolution de l'azithromycine à l'aide d'acide chorhydrique. Le pH de la solution est ajusté à 7,1 avec du trishydroxyméthylaminométhane.

La solution est conservée à l'abri de la lumière dans des conditions de température et d'humidité respectivement de 40 °C et 75 %. Des échantillons sont prélevés de la solution à différents intervalles de temps et analysés par chromatographie liquide à haute pression afin d'observer la dégradation de l'azithromycine au cours du temps. Pour la solution aqueuse, on commence à observer une dégradation de la molécule après environ quatre semaines, qui s'accélère ensuite pour atteindre environ 80 % de composé dégradé après deux mois.

Par contre, en ce qui concerne la composition suivant l'invention, étudiée dans les mêmes conditions, l'azithromycine reste stable même après 6 mois de conservation. La solution reste limpide et transparente.

### Exemple 5

On réalise un collyre à base d'azithromycine à 0,5 % en poids, comportant un agent de conservation. Ce collyre, destiné à être conditionné en flaconnages multidoses, présente la composition suivante, exprimée en poids pour 100 g :

| | |
|---|---|
| Azithromycine | 0,5 g |
| Chlorure de benzalkonium | 0,01 g |
| Triglycérides à chaîne moyenne | Q.S.P. 100 g |

Une masse de triglycérides TCM correspondant à 99 % de sa masse finale normale est chauffée à 70 °C au bain-marie. Les poudres d'azithromycine ainsi que de chlorure de benzalkonium sont dissoutes dans le TCM sous agitation. L'ensemble est maintenu à 70 °C pendant quelques minutes, puis on le laisse refroidir jusqu'à température ambiante. On ajuste alors au poids avec le TCM, et on parfait le mélange quelques minutes sous agitation. La solution est ensuite filtrée sous ambiance stérile, sur un filtre de 0,2 µm de type à haut débit en membrane de polyéthersulfone.

### Exemple 6

En procédant comme dans les exemples précédents, on réalise un collyre à base de roxithromycine dosé à 0,5 % en poids. La composition comporte un agent de conservation, car elle est destinée à être conditionnée en flaconnages multidoses.

| | |
|---|---|
| Roxithromycine | 0,5 g |
| Chlorure de benzalkonium | 0,01 g |
| Triglycérides à chaîne moyenne | Q.S.P. 100 g |

### Exemple 7

On réalise un collyre à base de clarithromycine dosé à 0,5 % en poids, destiné à être conditionné en flaconnages multidoses.

La composition centésimale est la suivante :

| | |
|---|---|
| Clarithromycine | 0,5 g |
| Chlorobutanol | 0,5 g |
| Triglycérides à chaîne moyenne | Q.S.P. 100 g |

Une masse de triglycérides à chaîne moyenne du commerce (TCM) correspondant à 99 % de sa masse finale normale est chauffée à 70 °C au bain-marie. La poudre de clarithromycine est dissoute dans le TCM sous agitation. L'ensemble est maintenu à 70 °C pendant quelques minutes, puis on le laisse refroidir jusqu'à une température de 40 °C. On ajoute alors le chlorobutanol et on maintient l'agitation jusqu'à complet refroidissement. On ajuste le poids avec le TCM et on parfait le mélange quelques minutes sous agitation. La solution est finalement filtrée sous ambiance stérile, sur membrane de polyéthersulfone filtrant à 0,2 µm.

### Exemple 8

En procédant comme dans l'exemple précédent, on réalise un collyre à base d'érythromycine, contenant 0,5 % en poids d'érythromycine. Le collyre étant destiné à être conditionné en flaconnages unidoses, il n'est pas ajouté de conservateur.

### Exemple 9

On réalise un collyre à base d'érythromycine à 0,5 % en poids, répondant à la composition centésimale suivante :

| | |
|---|---|
| Erythromycine | 0,5 g |
| Chlorobutanol | 0,5 g |
| Triglycérides à chaîne moyenne | Q.S.P. 100 g |

On procède comme dans les exemples précédents. La solution obtenue est limpide. Sa viscosité est de l'ordre de 30 mPa.s à la température ordinaire. Le produit est commercialisé en flaconnages unidoses pour distribution goutte à goutte en application dans l'oeil.

En variante, dans cet exemple comme dans les précédents, la viscosité peut être modulée à des valeurs pouvant aller par exemple jusqu'à 100 mPa.s en ajoutant à la solution un épaississant miscible dans le TCM.

Des viscosités supérieures sont réalisables, mais on préfère ici conserver une fluidité suffisante pour que la solution convienne en collyre et que la formation des gouttes ne soit pas entravée**.**

### Exemple 10

On soumet un collyre préparé selon l'exemple 2 à des essais de pharmacocinétique pour examiner la biodisponibilité oculaire du médicament. Les dosages sont effectués dans la conjonctive, dans la cornée et dans les larmes des yeux de lapins en comparaison avec deux autres préparations à base d'azithromycine. Le taux d'azithromycine est mesuré plusieurs fois par jour pendant six jours après l'administration d'une dose initiale unique.

Trois formules sont ainsi testées. La première concerne une dose orale d'azithromycine concentrée à 20 mg/kg, administrée par absorption d'un comprimé. La seconde concerne une dose oculaire d'une suspension aqueuse d'azithromycine concentrée à 1 % en poids, administrée localement en instillant une goutte de la suspension à la surface de l'oeil du lapin. La troisième concerne la solution huileuse préparée conformément à l'exemple 2, qui est administrée de façon similaire.

Les résultats sur la conjonctive montrent que la première formule, à savoir la dose orale d'azithromycine, ne permet pas de dépasser une concentration conjonctivale en azithromycine supérieure à 0,1 µg/g, alors que les deux autres formules conduisent à des taux significatifs, 24 heures après l'administration, qui se situent à 0,55 µg/g pour la formule aqueuse et à 0,60 µg/g pour la formule huileuse de l'invention. La supériorité de cette dernière ressort surtout sur la prolongation de la présence d'un taux d'azithromycine moyen minimum. Ce taux est en effet supérieur à 0,25 µg/g (taux minimal pour éliminer les germes) pendant une journée seulement pour la forme en suspension aqueuse, alors que pour la solution huileuse (collyre préparé selon l'exemple 2) il reste supérieur à 0,25 µg/g sur une période de trois jours.

Concernant la cornée, la dose orale ne devient détectable qu'au bout de 72 heures pour atteindre 0,25 µg/g au bout de six jours. La suspension aqueuse conduit à des taux de l'ordre de 3 µg/g une journée après instillation qui diminue jusqu'à s'abaisser à 0,6 µg/g six jours après instillation. La solution huileuse se révèle bien supérieure ; la concentration en azithromycine atteint 3,8 µg/g 24 heures après l'instillation et reste supérieure à 1µg/g six jours après.

Outre le fait que l'azithromycine présente un très net tropisme cornéen, la formulation du collyre préparé selon l'exemple 2 est particulièrement adaptée à une délivrance optimale du principe actif dans cette zone oculaire. Dans le cas de la cornée, l'utilisation topique de l'azithromycine s'avère plus efficace pour délivrer rapidement le principe actif sur son site d'action.

Concernant les essais dans les larmes, les taux lacrymaux en azithromycine sont encore microbiologiquement significatifs une journée après instillation. Les taux lacrymaux sont de 1,9 µg/g pour la suspension aqueuse et de 2,0 µg/g pour la solution huileuse. Ils sont insuffisants pour la dose orale, la présence d'azithromycine n'étant pas détectable dans ce cas.

### Exemple 11

On soumet les formulations préparées suivant les exemples 1 et 4 à une étude de pharmacocinétique oculaire chez l'homme.

Les tests consistent à instiller une goutte de collyre sur la surface oculaire de patients à un ou plusieurs instants donnés, puis à effectuer des prélèvements de larmes sur les yeux traités à différents intervalles de temps.

Les échantillons prélevés sont analysés par chromatographie liquide à haute pression couplée à la spectrométrie de masse, afin de déterminer leur teneur en azithromycine. Les résultats ainsi obtenus correspondent à la concentration en azithromycine dans les larmes à chaque temps de prélèvement. Afin d'obtenir l'effet thérapeutique désiré, on vérifie que la concentration d'azithromycine dans les larmes soit au moins égale à 0,5 µg/g. Cette valeur correspond à la concentration minimale inhibitrice généralement décrite pour l'azithromycine comme étant la concentration minimale nécessaire pour obtenir une efficacité thérapeutique au niveau oculaire, pour l'ensemble des germes sensibles. Ainsi, la concentration en azithromycine et le temps de rémanence de la composition doivent être suffisamment élevés, en relation l'un avec l'autre, pour qu'une dose d'antibiotique supérieure ou égale à la concentration inhibitrice minimale (CIM) soit délivrée aux tissus oculaires visés.

### Cinétique lacrymale après administration d'une goutte unique de collyre :

Pour chaque formulation, une mesure de la concentration lacrymale d'azithromycine est effectuée à 0,5 ; 1 ; 2 ; 4 ; 8 et 24 heures après administration d'une goutte à la surface de l'oeil.

Pour le collyre dosé à 0,5 % en azithromycine, la concentration en azithromycine dans les larmes est inférieure à 0,85 µg/g, valeur correspondant au seuil minimal de quantification de la méthode de dosage, et ce pour tous les prélèvements, y compris pour ceux effectués très tôt, dans un délai d'une heure suivant l'administration.

En ce qui concerne le collyre dosé à 1,5 % en azithromycine, on obtient les concentrations lacrymales suivantes :

| **Temps (heures)** | **[Azithromycine] / Larmes (µg/g)** |
|---|---|
| 0,5 | 8,85 |
| 1 | 4,53 |
| 2 | 2,45 |
| 4 | 1,68 |
| 8 | 0,87 |
| 24 | 0,91 |

On peut noter ici qu'on retrouve encore de l'azithromycine dans les larmes, à un taux très largement supérieur à 0,5 µg/g, 24 heures après administration d'une dose unique de collyre à 1,5 %:

Des résultats satisfaisants ont également été observés pour une concentration du collyre à 1 % en azithromycine, une concentration de l'ordre de 1 à 1,5 % en azithromycine apparaissant ainsi appropriée pour délivrer une concentration thérapeutiquement efficace chez l'homme.

### Cinétique lacrymale après administration répétée du collyre :

Cette étude consiste à effectuer le dosage de l'azithromycine dans les larmes, comme précédemment, mais après administration de plusieurs gouttes de collyre à des intervalles de temps réguliers.

Le collyre est ici dosé à 1,5 % en azithromycine. Une première goutte est administrée au temps 0, puis une autre à chacun des temps suivants : 12 ; 24 ; 48 et 72 heures. Des prélèvements lacrymaux sont effectués 72 ; 96 ; 120 et 144 heures après la première administration.

Les concentrations suivantes d'azithromycine dans les larmes sont obtenues :

| **Temps (heures)** | **[Azithromycine] / Larmes (µg/g)** |
|---|---|
| 72 | 2,53* |
| 96 | 2,98 |
| 120 | 5,61 |
| 144 | 2,14 |

| | |
|---|---|
| * valeur obtenue avant administration de la goutte à 72 heures. | |

Après cinq applications successives de la solution d'azithromycine à 1,5 % de l'exemple 1, réparties sur une durée de 3 jours (soit 72 heures), on retrouve toujours une concentration d'azithromycine élevée dans les larmes 6 jours (soit 144 heures) après la première administration.

La description qui précède explique clairement, notamment à l'aide des exemples détaillés qui viennent d'être exposés, comment l'invention procède d'une manière que ne pouvait prédire l'homme de l'art pour atteindre les objectifs qu'elle s'est fixés. En particulier, il ressort clairement des résultats précédents, qu'un nombre limité d'instillations de la composition à 1 ou 1,5 % en azithromycine suivant l'invention, permet d'obtenir des concentrations lacrymales bien supérieures à la concentration inhibitrice minimale pendant une période relativement longue et que, par les profils pharmacocinétiques obtenus chez l'homme, elle est particulièrement appropriée pour le traitement des infections oculaires bactériennes par un régime d'administration constitué d'un faible nombre de doses.

Le fait qu'un petit nombre d'instillations soit seulement nécessaire pour obtenir la délivrance prolongée dans les tissus oculaires d'une dose d'antibiotique thérapeutiquement efficace offre de nombreux avantages, en particulier de confort de traitement, et de réduction du risque que l'utilisateur peut avoir de développer une sensibilisation à un des constituants de la composition. En d'autres termes, plus la durée du traitement est courte, et le nombre d'instillations limité, et plus le risque de réaction/irritation de l'oeil est réduit. L'utilisation topique présente l'avantage d'être potentiellement efficace au niveau antimicrobien dès l'application de la dose, contrairement à l'utilisation d'une forme orale. De plus, la formulation huileuse permet d'obtenir des taux lacrymaux, conjonctivaux et cornéens supérieurs à ceux obtenus à partir de suspensions aqueuses.

Ces avantages dans l'efficacité antibiotique en traitement des maladies oculaires viennent s'ajouter à ceux qui sont plutôt liés à la forme physique de solution par distinction avec les suspensions connues ou aux conditions de fabrication, notamment en ce qui concerne la commodité de la stérilisation. On notera en plus que l'invention se prête à une mise en oeuvre des composés actifs de la famille des macrolides sous leur forme base plutôt que sous la forme saline, qui serait inévitable pour une solution aqueuse, à laquelle on devrait alors ajouter des tampons d'ajustement du pH.

Enfin, l'utilisation des triglycérides d'acides gras suivant l'invention est particulièrement avantageuse dans le cadre d'une utilisation en application locale sur les tissus oculaires, car elle permet d'obtenir un temps de rémanence important sur la surface de l'oeil, tout en maintenant un risque de réaction inflammatoire réduit au minimum, du fait de la composition chimique bien définie des triglycérides d'acides gras et de leur bonne capacité de solubilisation de l'ensemble du composé actif, sans nécessiter pour autant l'ajout de composés additionnels.

## Revendications

1. Composition pharmaceutique de traitement d'infections oculaires par application locale, **caractérisée en ce qu'**elle se présente sous la forme d'un collyre contenant comme agent thérapeutiquement actif un composé antibiotique de la classe des macrolides choisi parmi les azalides, en solution dans un véhicule huileux essentiellement constitué de triglycérides d'acides gras.

2. Composition pharmaceutique selon la revendication 1, **caractérisée en ce que** ledit véhicule huileux est une huile grasse d'origine végétale reconnue physiologiquement compatible et essentiellement constituée de triglycérides d'acides carboxyliques d'hydrocarbures saturés à chaîne linéaire de longueur moyenne.

3. Composition pharmaceutique selon la revendication 1 ou 2, **caractérisée en ce que** ledit véhicule huileux est essentiellement constitué de triglycérides d'acides gras dont la molécule comporte de 5 à 12 atomes de carbone, et de préférence de 8 à 10 atomes de carbone comme l'acide caprylique et/ou l'acide caprique.

4. Composition pharmaceutique selon l'une quelconque des revendications précédentes, **caractérisée en ce que** ledit véhicule huileux présente une viscosité d'au moins 10 mPa.s et d'au plus 100 mPa.s à la température ordinaire, ladite viscosité étant notamment comprise entre 10 et 50 mPa.s, et plus particulièrement de l'ordre de 20 à 40 mPa.s, et **en ce que** ledit véhicule huileux est de densité proche de 1, notamment comprise entre 0,9 et 1.

5. Composition pharmaceutique selon l'une quelconque des revendications précédentes, **caractérisée en ce que** ledit véhicule huileux possède un indice de réfraction compris entre 1 et 2, préférentiellement compris entre 1,3 et 1,5, notamment de l'ordre de 1,45.

6. Composition pharmaceutique selon l'une quelconque des revendications précédentes, **caractérisée en ce qu'**elle est présentée en flaconnages unidoses.

7. Composition selon l'une quelconque des revendications 1 à 6, **caractérisée en ce que** ledit agent thérapeutiquement actif est l'azithromycine, à une concentration avantageusement comprise entre 0,7 et 2 % en poids, et préférentiellement entre 1 et 1,5 % en poids.

8. Procédé de préparation d'une composition pharmaceutique de collyre convenant pour application locale en ophtalmologie, **caractérisé en ce qu'**il consiste à mettre un composé antibiotique de la classe des macrolides choisi parmi les azalides, et plus particulièrement l'azithromycine, en solution dans un véhicule huileux avantageusement d'origine végétale et/ou constitué de triglycérides, essentiellement des triglycérides d'acides gras à chaîne linéaire de longueur moyenne.

9. Procédé suivant la revendication 8, **caractérisé en ce que** ladite composition pharmaceutique est stérilisée par filtration au travers d'une membrane filtrante de dimensions de pores au plus égales à 0,2 µm, de préférence à la température ordinaire, éventuellement sous léger chauffage à une température restant inférieure à 80 °C.

10. Composition pharmaceutique sous forme de collyre pour le traitement d'infections oculaires par application locale, **caractérisée en ce qu'**elle contient comme agent thérapeutiquement actif de l'azithromycine en solution dans un véhicule huileux, à une concentration comprise entre 1 et 1,5 % en poids du poids total, ledit véhicule huileux étant essentiellement constitué de triglycérides d'acides carboxyliques d'hydrocarbures saturés à chaîne linéaire de longueur moyenne, notamment d'acides tels que l'acide caprylique et/ou l'acide caprique.

11. Utilisation de l'azithromycine sous forme de base et mise en solution dans un véhicule huileux pour la fabrication d'un collyre convenant au traitement d'infections oculaires par application locale sur l'oeil.

12. Composition pharmaceutique de traitement des infections oculaires par application locale, **caractérisée en ce qu'**elle se présente sous la forme d'un collyre contenant comme agent thérapeutiquement actif de l'azithromycine en solution dans un véhicule huileux essentiellement constitué de triglycérides d'acides gras, à une concentration comprise entre 0,7 et 2% en poids.

## Claims

1. A pharmaceutical composition for treating eye infections by local application, **characterized in that** it is under the form of an eye lotion that contains as therapeutically active agent an antibiotic compound from the class of azalides which is dissolved in an oily vehicle essentially made of fatty acid triglycerides.

2. A pharmaceutical composition according to claim 1, **characterized in that** said oily vehicle is fatty oil of plant origin that is known as being physiologically compatible and consists essentially of triglycerides of carboxylic acids from saturated hydrocarbons having a linear chain of medium length.

3. A pharmaceutical composition according to claim 1 or 2, **characterized in that** said oily vehicle is essentially made of triglycerides of fatty acids the molecule of which comprises from 5 to 12 carbon atoms. and preferably from 8 to 10 carbon atoms, such as caprylic acid and/or capric acid.

4. A pharmaceutical composition according to any of the preceding claims, **characterized in that** said oily vehicle shows a viscosity of at least 10 mPa.s and not more than 100 mPa.s at nomal temperature, typically from 20 to 40 mPa.s, and **in that** said oily vehicle has a density close to 1, especially from 0.9 to 1.

5. A pharmaceutical composition according to any of the preceding claims, **characterized in that** said oily vehicle-shows a refractive index from 1.3 to 1.5, typically about 1.45.

6. A pharmaceutical composition according to any of the above claims, **characterized in that** it is presented in single-dose eye-drop bottles.

7. A composition according to any of claims 1 to 6, **characterized in that** said therapeutically active compound is azithromycin, at a concentration advantageously within the range from 0.7 to 2 % by weight, and preferably from 1 to 1.5 % by weight.

8. A process for preparing a pharmaceutical eye lotion composition suitable for local application in ophthalmology, **characterized in that** it comprises dissolving an antibiotic compound from the class of macrolides selected from azalides, and more specifically azithromycin, in an oily vehicle that is advantageously of plant origin and/or composed of triglycerides, essentially triglycerides of fatty acids with a medium-chained linear chain.

9. A process according to claim 8, **characterized in that** said pharmaceutical composition is sterilized by filtration through a filtering membrane with a pore size not higher than 0.2 µm, preferably at normal temperature, or possibly undergentle hating up to a temperature not higher than 80°C.

10. A pharmaceutical composition under the forme of an eye-drop solution for treating eye infections by local application, **characterized in that** it contains as a therapeutically active agent an azithromycin solution in an oily vehicle, at a concentration advantageously within the range from 1 to 1.5 % by weight with respect to the total composition weight, wherein said oily vehicle consists essentially of triglycerides of carboxylic acids from saturated hydrocarbons with a linear chain of medium length, especially such acids as caprylic acid and/or capric acid.

11. The use of azithromycin in base form and dissolved in an oily vehicle for producing an eye-drop lotion suitable for the treatment of eye infections by local application onto the eye.

12. A pharmaceutical composition for the treatment of eye infections by local application, **characterized in that** it is under the form of an eye-drop lotion that contains, as therapeutically active agent, a solution of azithromycin in an oily vehicle essentially made of fatty acid triglycerides, at a concentration from 0.7 to 2 % by weight.

## Patentansprüche

1. Pharmazeutisches Mittel zur Behandlung von Augeninfektionen durch lokale Anwendung, **dadurch gekennzeichnet, dass** es in Form von Augentropfen vorliegt, die in einer im Wesentlichen aus Fettsäuretriglyceriden bestehenden öligen Grundlage als Wirkstoff ein unter den Azaliden ausgewähltes Makrolid-Antibiotikum gelöst enthalten.

2. Pharmazeutisches Mittel nach Anspruch 1, **dadurch gekennzeichnet, dass** die ölige Grundlage ein fettes Öl pflanzlichen Ursprungs ist, welches anerkanntermaßen physiologisch kompatibel ist und im Wesentlichen aus Triglyceriden gesättigter geradkettiger Carbonsäuren mit mittlerer Länge besteht.

3. Pharmazeutisches Mittel nach Anspruch 1 oder 2, **dadurch gekennzeichnet, dass** die ölige Grundlage im Wesentlichen aus Triglyceriden von Fettsäuren, deren Molekül 5 bis 12 Kohlenstoffatome und vorzugsweise 8 bis 10 Kohlenstoffatome aufweist, wie Caprylsäure und/oder Caprinsäure, besteht.

4. Pharmazeutisches Mittel nach einem der vorhergehenden Ansprüche, **dadurch gekennzeichnet, dass** die ölige Grundlage eine Viskosität von mindestens 10 mPa.s und höchstens 100 mPa.s bei Normaltemperatur aufweist, wobei die Viskosität insbesondere in einem Bereich von 10 bis 50 mPa.s und vor allem in der Größenordnung von 20 bis 40 mPa.s liegt und die ölige Grundlage eine Dichte von nahezu 1, insbesondere von 0,9 bis 1 aufweist.

5. Pharmazeutisches Mittel nach einem der vorhergehenden Ansprüche, **dadurch gekennzeichnet, dass** die ölige Grundlage einen Refraktionsindex im Bereich von 1 bis 2, vorzugsweise von 1,3 bis 1,5, insbesondere in der Größenordnung von 1,45 aufweist.

6. Pharmazeutisches Mittel nach einem der vorhergehenden Ansprüche, **dadurch gekennzeichnet, dass** es in Einzeldosis-Behältnissen konfektioniert ist.

7. Mittel nach einem der Ansprüche 1 bis 6, **dadurch gekennzeichnet, dass** der Wirkstoff Azithromycin ist, dessen Konzentration vorteilhafterweise in einem Bereich von 0,7 bis 2 Gew.-% und vorzugsweise in einem Bereich von 1 bis 1,5 Gew.-% liegt.

8. Verfahren zur Herstellung eines pharmazeutischen Mittels in Form von Augentropfen, die zur lokalen Anwendung in der Ophtalmologie geeignet sind, **dadurch gekennzeichnet, dass** man in einer öligen Grundlage, die vorteilhafterweise pflanzlichen Ursprungs ist und/oder aus Triglyceriden, im Wesentlichen aus Triglyceriden geradkettiger Fettsäuren mit mittlerer Kettenlänge besteht, ein unter den Azaliden ausgewähltes Makrolid-Antibiotikum, insbesondere Azithromycin, löst.

9. Verfahren nach Anspruch 8, **dadurch gekennzeichnet, dass** man das pharmazeutische Mittel sterilisiert, indem man es durch eine Filtermembran mit Porengrößen von höchstens 0,2 µm vorzugsweise bei Normaltemperatur, gegebenenfalls unter leichtem Erwärmen auf eine Temperatur unterhalb von 80 °C, filtriert.

10. Pharmazeutisches Mittel in Form von Augentropfen zur Behandlung von Augeninfektionen durch lokale Anwendung, **dadurch gekennzeichnet, dass** es in einer öligen Grundlage als Wirkstoff Azithromycin mit einer Konzentration im Bereich von 1 bis 1,5 Gew.-%, bezogen auf das Gesamtgewicht, gelöst enthält, wobei die ölige Grundlage im Wesentlichen aus Triglyceriden gesättigter geradkettiger Carbonsäuren mit mittlerer Kettenlänge, insbesondere von Säuren wie Caprylsäure und/oder Caprinsäure, besteht.

11. Verwendung von Azithromycin in Form der Base und in einer öligen Grundlage gelöst zur Herstellung von Augentropfen, die zur Behandlung von Augeninfektionen durch lokale Anwendung am Auge geeignet sind.

12. Pharmazeutisches Mittel zur Behandlung von Augenerkrankungen durch lokale Anwendung, **dadurch gekennzeichnet, dass** es in Form von Augentropfen vorliegt, die in einer öligen Grundlage, die im Wesentlichen aus Fettsäuretriglyceriden besteht, als Wirkstoff Azithromycin mit einer Konzentration im Bereich von 0,7 bis 2 Gew.-% enthalten.
